# EUROPÄISCHE PATENTANMELDUNG

(11) **EP 3 892 118 A1**
(43) Veröffentlichungstag der Anmeldung: **13.10.2021**
(21) Anmeldenummer: 21166880.1
(22) Anmeldetag: 06.04.2021
(51) Int. Cl.: A23L 33/15, A23L 33/165, A23L 33/19, A61K 31/375, A61K 33/26, A61K 36/738, A61K 36/75, A61K 38/40, A61P 1/00, A61P 3/02

(54) **EISEN-ENTHALTENDE FORMULIERUNG, ERNÄHRUNGSPHYSIOLOGISCHE ZUSAMMENSETZUNG, WELCHE DIESE ENTHÄLTE, UND VERWENDUNG DERSELBEN**

(30) Priorität: 06.04.2020 DE 202020101861 U
(71) Anmelder: artgerecht GmbH, 60313 Frankfurt (DE)
(72) Erfinder: Baum, Matthias, 22763 Hamburg (DE); Reheis, Daniel, 60329 Frankfurt am Main (DE)
(74) Vertreter: K & H Bonapat Patentanwälte Koch · von Behren & Partner mbB

(57) **Zusammenfassung**

Die Erfindung betrifft eine Formulierung, die Lactoferrin, Eisenverbindungen und Vitamin C umfasst. Sie betrifft ferner eine ernährungsphysiologische Zusammensetzung mit dieser Formulierung und eine magensäurestabile Kapsel mit dieser Formulierung oder dieser ernährungsphysiologischen Zusammensetzung. Schließlich umfasst die Erfindung eine Verwendung der Formulierung oder ernährungsphysiologischen Zusammensetzung für eine ergänzend bilanzierte Diät zur Anwendung bei einem erhöhten Eisenbedarf bei entzündlichen Darmerkrankungen und Unverträglichkeit von Eisensalzen.

## Beschreibung

Die vorliegende Erfindung betrifft das Diätmanagement bei erhöhtem Bedarf an Eisen bei chronisch entzündlichen Darmerkrankungen und Unverträglichkeit von Eisensalzen. Insbesondere betrifft die vorliegende Erfindung eine Eisen-enthaltende Formulierung, eine ernährungsphysiologische Zusammensetzung, welche diese enthält, und die Verwendung derselben für eine ergänzend bilanzierte Diät zur Anwendung bei einem erhöhten Eisenbedarf bei chronisch entzündlichen Darmerkrankungen und Unverträglichkeit von Eisensalzen.

Eisen ist ein essentielles Spurenelement, das der Körper nicht selber produzieren kann. Es ist vor allem für die Bildung des roten Blutfarbstoffes Hämoglobin und damit den Sauerstofftransport wichtig. Aber auch als Bestandteil der Energieerzeugung in den Zellen und von Enzymen ist Eisen von großer Bedeutung für den Körper, sowie auch für die DNA-Replikation. Der menschliche Körper enthält etwa 2 bis 4 Gramm Eisen. Ungefähr 60 Prozent davon sind an den roten Blutfarbstoff Hämoglobin, die restlichen 40 Prozent an Ferritin, Hämosiderin, Myoglobin und Enzyme gebunden. Der Bedarf an Eisen pro Tag ergibt sich aus den täglichen Eisenverlusten über Schweiß, Urin und Stuhl und beträgt zwischen einem und zwei Milligramm. Frauen verlieren zusätzlich Eisen während ihrer Periode.

Ein Eisenmangel gehört zu den häufigsten Mangelerscheinungen weltweit, wobei etwa 30 Prozent, also über zwei Milliarden Menschen, insbesondere Frauen, davon betroffen sind.

Die häufigsten Ursachen des Eisenmangels sind regional unterschiedlich: In westlichen Industrieländern sind chronische Blutverluste, beispielsweise durch konsumierende Prozesse wie Tumorerkrankungen, Hypermenorrhoe oder chronische entzündliche Prozesse, wie z.B. Osteomyelitis, vorherrschend, während in der dritten Welt bakterielle (z.B. Tuberkulose), parasitäre (z.B. Malaria) und auf eine Mangelernährung zurückzuführende Ursachen dominieren.

Einige verbreitete Symptome und Folgeerkrankungen eines Eisenmangels sind z.B. Blässe, Müdigkeit, Schwindelgefühl, Kopfschmerzen, Konzentrationsstörungen, Eisenmangelanämie und Herzinsuffizienz.

Wenn eine Fehl- oder Mangelernährung die Ursache eines Eisenmangels ist, sollten mehr eisenhaltige Nahrungsmitteln aufgenommen werden, wobei die Eisenzufuhr je nach Alter und Geschlecht 10 bis 12 mg/Tag betragen sollte.

Falls der Eisenmangel sehr ausgeprägt ist oder nicht allein durch Ernährungsumstellung ausgeglichen werden kann, können Eisenpräparate in Tablettenform (peroral) oder als Infusion verabreicht werden. Eine perorale Verabreichung ist meist vorzuziehen, da dies dem natürlichen Weg entspricht, auf dem Eisen durch den Körper aufgenommen wird.

Eine Eisen-Mangelernährung ist beispielsweise bei einer entzündlichen Darmerkrankung (IBD, Inflammatory Bowel Disease) (z.B. Morbus Crohn (CD) und Colitis ulcerosa (UC, Ulcerative Colitis) verbreitet. Eine IBD ist ein lebenslang rezidivierender und remittierender Zustand, der durch eine Entzündung des Darms gekennzeichnet ist. Die Häufigkeit eines Eisenmangels (ID) in Patienten mit IBD beträgt etwa 45 % (vgl. L. Peyrin-Biroulet et al., Am. J. Clin. Nutr., 102(6), 1585-1594, 2015). Mehrere Gründe tragen zur einer Eisen-Mangelernährung und einem Eisenmangel in dieser Patientenpopulation bei, wie z.B. eine unzureichende Eisenaufnahme mit der Nahrung, ein Blutverlust von entzündeter Schleimhaut und eine beeinträchtigte Absorption z.B. aufgrund einer Dünndarmentzündung oder einer Darmresektion (vgl. A. D. de Silva et al., Inflamm. Bowel Dis. 9(5), 316-320, 2003).

Eine chronische Mangelernährung bei IBD ist auf die verminderte Nahrungsgesamtaufnahme aufgrund von Schmerzen und Müdigkeit zurückzuführen, sowie auf die Vermeidung von Nahrungsmittelgruppen, welche die Symptome verstärken (vgl. K. Kilby et al., Nutrients 11(6), 2019).

Eine Schleimhautblutung führt zu einem Eisenverlust über den Stuhl, wodurch der Bedarf für eine Nahrungsergänzung von Eisen erhöht wird. Die Entzündungsprozesse bei IBD verändern jedoch die hepatische Hepcidin-Expression und somit die Eisenabsorption und den Eisenmetabolismus.

Ferner kann eine elektive Chirurgie den Mikroernährungsstatus weiter verschlechtern, was das Ausmaß der Schleimhautoberfläche vermindert, die für eine Absorption zur Verfügung steht (vgl. A. J. Lucendo et al., World J. Gastroenterol. 15(17), 2009).

Der Eisenmangel wird sich schließlich zu einer Eisenmangelanämie (IDA) entwickeln, welche die Lebensqualität und das Arbeitsvermögen durch Beeinträchtigen der physischen, emotionalen und kognitiven Funktionen signifikant verschlechtern kann. Diagnostische Kriterien für einen Eisenmangel hängen von dem Niveau der Entzündung ab, wie es in der nachstehenden Tabelle 1 gezeigt ist (vgl. C. Gasche et al., Inflamm. Bowel Dis. 13(12), 1545-1553, 2007).

**Tabelle 1: Ausmaß des Eisenmangels, der durch Serum-Ferritin oder die Transferrin-Sättigung in Erwachsenen bewertet wird**

| | Serum-Ferritin (µg/L) | Transferrin-Sättigung (%) |
|---|---|---|
| Abgereicherte Eisenkonzentration in gesunden Erwachsenen oder Patienten mit ruhender IBD | <30 | <16 |
| Abgereicherte Eisenkonzentration während einer aktiven IBD | <100 | <16 |
| Angemessene Eisenkonzentration in gesunden Personen | >100 | 16 bis 50 |
| Potenzielle Eisenüberversorgung | >800 | >50 |

Patienten, die an IBD leiden, versuchen üblicherweise, ihre Krankheit dadurch zu beherrschen, dass sie eine Diät einhalten, die alle Nahrungsmittel vermeidet, welche die Symptome auslösen könnten. Daher ist eine Erhöhung der Eisenaufnahme durch Nahrungsmittel, z.B. durch eine Erhöhung des Konsums von Blattgemüse oder Fleisch, gewöhnlich nicht möglich (vgl. J. K. Limdi et al., Inflamm. Bowel Dis. 22(1), 164-170, 2016).

Die Erhöhung der Eisenaufnahme durch Nahrungsmittel über Nahrungsergänzungsmittel ist häufig unmöglich, da die Eisensalze, die sie üblicherweise enthalten, schlecht verträglich sind und andere Krankheiten auslösen können (z.B. eine Geschwürbildung, das Wachstum von pathogenen Bakterien). Die sichereren Eisen(III)-Salze sind schlecht bioverfügbar, insbesondere wenn eine zugrundeliegende Entzündung die Redox-Umwandlung von Eisen(III) zu Eisen(II) verschlechtert, die eine Voraussetzung für die Eisenabsorption ist (vgl. C. Gasche et al., Inflamm. Bowel Dis. 13(12), 1545-1553, 2007).

Wenn IBD-Patienten ihren Eisenbedarf mit ihrer Ernährung nicht decken können, sind häufig Eiseninfusionen erforderlich, die für die Patienten unbequem sind und ebenfalls die vorstehend genannten Nachteile aufweisen.

Es ist daher eine Aufgabe der vorliegenden Erfindung, für Patienten, die an entzündlichen Darmerkrankungen und einer Unverträglichkeit von Eisensalzen leiden, einen Weg zur Verabreichung von Eisen zu finden, der die vorstehend genannten Nachteile vermeidet.

Gemäß der vorliegenden Erfindung werden eine Eisen-enthaltende Formulierung, eine ernährungsphysiologische Zusammensetzung, welche diese enthält, und die Verwendung derselben für eine ergänzend bilanzierte Diät zur Anwendung bei einem erhöhten Eisenbedarf bei entzündlichen Darmerkrankungen und Unverträglichkeit von Eisensalzen bereitgestellt. Die vorliegenden Erfinder haben gefunden, dass eine Formulierung, die Lactoferrin, Eisenverbindungen und Vitamin C umfasst, die vorstehend genannten Nachteile des Standes der Technik beseitigen kann.

Als Ergebnis dieser Erkenntnis stellt die vorliegende Erfindung eine Formulierung bereit, die Lactoferrin, Eisenverbindungen und Vitamin C umfasst.

Lactoferrin ist ein Protein (ein Transferrin, das dem Transport von Eisen dient), das in Säugetieren vorkommt und diverse Enzymaktivitäten aufweist, wie z.B. als Peptidase, und auch als Eisen-bindendes Protein wirkt. Es bietet somit eine Möglichkeit, Eisen in den menschlichen Körper einzubringen, das nicht als freies Eisen vorliegt und somit auch nicht die typischen nachteiligen Effekte von z.B. Eisen(III) mit sich bringt, die bei einer herkömmlichen Eisenzufuhr über Nahrungsergänzungsmittel auftreten (vgl. die vorstehende Diskussion). Lactoferrin dient auch selbst als Eisenquelle mit einem Eisengehalt von etwa 75 µg pro 100 mg Lactoferrin. Es kann über humane Lactoferrin-Rezeptoren im Darm aufgenommen werden. Ferner reguliert Lactoferrin die Bildung von Hydroxylradikalen, die während der Fenton-Reaktion gebildet werden und zum programmierten Zelltod führen, wobei dies auch eine der Ursachen zu sein scheint, warum bei der Verwendung von Formulierungen mit freiem Eisen vermehrt Nebenwirkungen auftreten. Lactoferrin sorgt so als Chelator (antioxidative Wirkung) für eine Pufferung der freien Radikalbildung, da es Eisen(III) aufnehmen kann. Systemisch weist Lactoferrin immunmodulierende und entzündungshemmende Eigenschaften auf, wobei es die direkte Entzündungsantwort reguliert, wodurch die proinflammatorische Zytokin-Menge abnimmt. Durch die regulierte Entzündungssituation wird auch die Hepcidin-Synthese vermindert, was die Verteilung von Eisen aus Enterocyten verbessert und die Verteilungsstörung reguliert.

Eisenverbindungen sind solche, wie sie z.B. in Pflanzen oder Tieren vorkommen, und umfassen keine herkömmlich verwendeten Eisensalze für eine Eisentherapie, die dem Fachmann bekannt sind, wie z.B. Eisencarbonat, Eisencitrat, Eisenammoniumcitrat, Eisenglukonat, Eisenfumarat, usw.

Ein Beispiel für solche Eisenverbindungen ist Ferritin. Ferritin ist ein Proteinkomplex, der als Eisenspeicher in Tieren, Pflanzen und Bakterien dient. Strukturell sind Ferritine scheibenförmige Proteine mit einer Größe im Nanometerbereich, die mit Eisenhydroxidoxid beladen sind. Ferritin kann über die menschlichen Enterocyten über einen separaten Rezeptorweg aufgenommen werden und umgeht so die UCP1- und HTM1-Rezeptorwege, die für freies Eisen (Fe(II)) und Häm-Eisen für die Aufnahme zuständig sind. Diese Rezeptorwege können durch Entzündungsprozesse, eine Darmmikrobiom-Dysbiose oder Polymorphismen des DMT1-Rezeptors eine mangelnde Funktionsfähigkeit aufweisen.

Vitamin C (Ascorbinsäure) nimmt eine aktive Rolle in der Eisenabsorption im menschlichen Körper, insbesondere bei nicht-Häm-Eisenquellen, ein. Ferner wird durch Vitamin C die Transferrin-vermittelte Eisenaufnahme erhöht. Da Ascorbinsäure wenig hitzebeständig ist, ist die separate Einnahme für eine verbesserte Eisenaufnahme sinnvoll. Nachweislich kann durch Ascorbinsäure die freie Eisenmenge von Eisen(II) erhöht werden. Als direktes Antioxidans werden freie Radikale gepuffert: Da nur Eisen(II) über den DMT1-Rezeptor aufgenommen werden kann, muss Eisen(III) im Darm zunächst in Eisen(II) umgewandelt werden, was ebenfalls zur Radikalbildung führt und im Darmlumen eine Dysbiose begünstigen kann.

Die Erfinder haben dabei gefunden, dass die vorstehend genannten Eisenverbindungen, insbesondere pflanzliche Eisenverbindungen, wie z.B. pflanzliches Ferritin, eine Menge an Eisen für den menschlichen Körper bereitstellen, die über einen separaten Rezeptorweg über Enterozyten aufgenommen werden kann. Grundsätzlich verbessert dabei das Vitamin C durch antioxidative Eigenschaften und Auswirkungen auf Transferrine die Aufnahme/Bioverfügbarkeit des Eisens. Lactoferrin kann als natürlicher Transporter für Eisen das zugeführte Eisen in Form der Eisenverbindungen und bestehendes Eisen in der Zelle an Zielorte im gesamten Organismus verteilen. Gleichzeitig dient Lactoferrin als Chelator, um die Bildung eines Überschusses an freien Radikalen zu reduzieren, was für einen funktionsfähigen Eisenstoffwechsel notwendig ist. Nicht zuletzt hat Lactoferrin systemische Auswirkung auf Entzündungssituationen: Durch die vermittelte entzündungshemmende Aktivität von Lactoferrin werden Entzündungszytokine (insbesondere Interleukin 6, IL6) reduziert, was im systemischen Kontext die Produktion von Hepcidin reduziert. Hepcidin hat einen direkten Einfluss auf den Ferroportinkanal, der für die Verteilung von Eisen aus Darm-Enterozyten verantwortlich ist. Durch das Vorliegen von weniger Hepcidin kann Eisen wieder (besser) im Körper verteilt werden. Auf diese Weise nimmt die vorliegende Formulierung in synergistischer Weise Einfluss auf unterschiedliche Mechanismen des Eisenhaushaltes, und zwar unabhängig davon, ob ein erhöhter Bedarf von Eisen, Verlust, Verbrauch, Mangel oder eine entzündungsbedingte Situation ursächlich ist.

Gemäß einer bevorzugten Ausführungsform wird das in der Formulierung verwendete Lactoferrin aus Kuhmilch oder Molke gewonnen. Verfahren zur Gewinnung von Lactoferrin aus Kuhmilch bzw. Molke sind dem Fachmann bekannt, vgl. z.B. EP 1 466 923 A1 oder EP 3 097 788 A1. Ferner ist Lactoferrin aus Kuhmilch bzw. Molke ein handelsübliches Produkt.

In einer besonders bevorzugten Ausführungsform umfasst das Lactoferrin, bezogen auf 100 Gew.-% des Lactoferrins, weniger als 0.00001, also 10^{∗}10^-6, Gew.-% Lactoperoxidase, weniger als 0.00001, also 10^{∗}10^-6, Gew.-% Angiogenin, und weniger als 0.00001, also 10^{∗}10^-6, Gew.-% Lipopolysaccharide. Dies entspricht 10^{∗}10^-8 g/g Lactoferrin. Handelsübliches Lactoferrin, das diese Substanzen enthält und daher keine hohe Qualität aufweist, kann Entzündungsprozesse sogar begünstigen und sich negativ auf die Eisen-Laborparameter auswirken. Gemäß der vorliegenden Erfindung ist es daher bevorzugt, ein aufgereinigtes/purifiziertes Lactoferrin zu verwenden, das möglichst geringe Konzentrationen von Lactoperoxidase, Angiogenin und Lipopolysacchariden aufweist, und das ganz besonders bevorzugt im Wesentlichen frei von diesen Substanzen gemäß den oben genannten Konzentrationen ist. Ein geeignetes Verfahren zur Herstellung eines solchen aufgereinigten Lactoferrins ist nachstehend in den Ausführungsbeispielen beschrieben.

Gemäß einer bevorzugten Ausführungsform handelt es sich bei den Eisenverbindungen um pflanzliche Eisenverbindungen. Pflanzliche Eisenverbindungen sind ganz besonders gut für die Aufnahme über einen separaten Rezeptorweg über Enterozyten geeignet.

Gemäß einer bevorzugten Ausführungsform liegen die pflanzlichen Eisenverbindungen in der Form eines Curryblattextrakts vor. Geeignete Curryblattextrakte sind handelsüblich.

Gemäß einer bevorzugten Ausführungsform liegt das Vitamin C in der Form eines Hagebuttenextrakts vor. Geeignete Hagebuttenextrakte sind handelsüblich.

Gemäß einer bevorzugten Ausführungsform umfasst die Formulierung gemäß der vorliegenden Erfindung, bezogen auf 100 Gew.-% der Formulierung, 70 bis 90 Gew.-% Lactoferrin, 1,5 bis 3,5 Gew.-% Eisenverbindungen und 6,5 bis 28,5 Gew.-% Vitamin C, mehr bevorzugt 75 bis 85 Gew.-% Lactoferrin, 1,7 bis 3,3 Gew.-% Eisenverbindungen und 10 bis 24 Gew.-% Vitamin C, noch mehr bevorzugt 77 bis 83 Gew.-% Lactoferrin, 1,9 bis 3,1 Gew.-% Eisenverbindungen und 15 bis 20 Gew.-% Vitamin C. Beispielsweise liegen in der Formulierung 100 bis 200 mg Lactoferrin, 3 bis 6 mg Eisenverbindungen und 15 bis 50 mg Vitamin C vor.

Gemäß einer bevorzugten Ausführungsform umfasst die Formulierung gemäß der vorliegenden Erfindung, bezogen auf 100 Gew.-% der Formulierung, 20 bis 30 Gew.-% Lactoferrin, 55 bis 75 Gew.-% Curryblattextrakt und 5 bis 15 Gew-% Hagebuttenextrakt, mehr bevorzugt 22 bis 28 Gew.-% Lactoferrin, 60 bis 70 Gew.-% Curryblattextrakt und 7 bis 13 Gew-% Hagebuttenextrakt, noch mehr bevorzugt 24 bis 26 Gew.-% Lactoferrin, 63 bis 68 Gew.-% Curryblattextrakt und 9 bis 11 Gew-% Hagebuttenextrakt.

Die vorliegende Erfindung stellt ferner eine ernährungsphysiologische Zusammensetzung bereit, die eine Formulierung, wie sie vorstehend beschrieben ist, und einen oder mehrere verträgliche(n) Hilfsstoff(e) umfasst.

Als verträgliche(n) Hilfsstoff(e) kann oder können übliche Hilfsstoffe verwendet werden, die dem Fachmann bekannt sind. Beispiele dafür umfassen Füllstoffe (z.B. Lactose, Cellulose, Stärke, Saccharose), Trennmittel (z.B. Siliziumdioxid), Bindemittel (z.B. Xanthan, Carrageen, Pektin, Traganth), Antioxidantien (Butylhydroxytoluol, α-Tocopherol), Konservierungsstoffe (Benzylalkohol, Benzalkoniumchlorid), Süßungsmittel (z.B. Saccharose, Sorbit), usw.

Die Anteile der vorliegenden Formulierung und des verträglichen Hilfsstoffs oder der verträglichen Hilfsstoffe in der ernährungsphysiologischen Zusammensetzung sind nicht speziell beschränkt und werden je nach der Verabreichungsform und dem Verabreichungszweck vom Fachmann in einer geeigneten Weise eingestellt.

Die vorliegende Erfindung stellt ferner eine magensäurestabile Kapsel, die eine Formulierung, wie sie vorstehend beschrieben ist, oder eine ernährungsphysiologische Zusammensetzung, wie sie vorstehend beschrieben ist, umfasst.

Die magensäurestabile Kapsel bewirkt, dass die Formulierung oder die ernährungsphysiologische Zusammensetzung erst nach dem Passieren des Magens im Zwölffingerdarm oder im weiteren Verlauf des Darms freigesetzt wird und somit eine Unverträglichkeitsreaktion mit der sensiblen Magenschleimhaut vermieden wird. Als Material für die magensäurestabile Kapsel wird vorzugsweise Hydroxypropylmethylcellulose verwendet.

Die vorliegende Erfindung stellt ferner die Verwendung einer Formulierung, wie sie vorstehend beschrieben ist, oder einer ernährungsphysiologischen Zusammensetzung, wie sie vorstehend beschrieben ist, für eine ergänzend bilanzierte Diät zur Anwendung bei einem erhöhten Eisenbedarf bei entzündlichen Darmerkrankungen und Unverträglichkeit von Eisensalzen bereit. Die vorliegende Verwendung stellt die Vorteile gegenüber herkömmlichen Eisentherapien bereit, wie sie vorstehend erläutert worden sind.

Die vorliegende Erfindung wird nachstehend mittels Ausführungsbeispielen, die in keinerlei Hinsicht beschränkend aufzufassen sind, näher erläutert.

### 1. Prozessbeschreibung zur Herstellung von purifiziertem Lactoferrin

Als Ausgangsprodukt für die Herstellung von purifiziertem Lactoferrin wird Laktenin aus Molke verwendet. Durch lonenaustauschchromatographie wird die schrittweise Auftrennung der Lakteninbestandteile erreicht. Die vier Pufferlösungen bestehen jeweils aus einer Mischung von Natriumacetat, Natriumchlorid, Essigsäure und Natronlauge in veränderlichen Verhältnissen. Der Rohstoff Laktenin wird an ein spezifisches lonenaustauscherharz gebunden. Durch Zufuhr des ersten Puffers können grobe Verunreinigungen gezielt gelöst und so aus dem Produkt entfernt werden. Mittels des zweiten Puffers wird ein weiteres Milchprotein, die Lactoperoxidase, gelöst, spezifisch abgetrennt und kann im weiteren Verfahren weiter aufbereitet werden. Mit der Spülung durch den dritten Puffer können Endotoxine und Angiogenin aus der Lösung entfernt werden. Der vierte Puffer dient zur spezifischen Abtrennung des Lactoferrins aus der Lösung und wird in weiteren Filtrationsprozessen aufkonzentriert, gefriergetrocknet und vermahlen.

### 2. Herstellung einer magensäurestabilen Kapsel, welche die erfindungsgemäße ernährungsphysiologische Zusammensetzung enthält

Curryblattextrakt, Lactoferrin, Hagebuttenextrakt und Siliziumdioxid als Trennmittel werden in einem Gewichtsverhältnis von 272,73:100,00:44,44 mit Siliziumdioxid als Trennmittel eingewogen und mit einem Mischer gemischt. Aus dem Gemisch wurden unter Verwendung von transparenten Hydroxypropylmethylcellulose-Kapseln mit einer vollautomatischen Kapselfüllmaschine magensäurestabile Kapseln hergestellt. Die Kapseln werden so befüllt, dass sie jeweils 272,73 mg Curryblattextrakt, 100,00 mg Lactoferrin, 44,44 mg Hagebuttenextrakt und 44,44 mg Siliziumdioxid enthielten.

### 3. Untersuchung der Wirksamkeit der erfindungsgemäßen ernährungsphysiologischen Zusammensetzung

Für eine Untersuchung der Wirksamkeit der erfindungsgemäßen ernährungsphysiologischen Zusammensetzung wurde die folgende Studie durchgeführt. Fünf gesunde Personen mit wurden für mindestens 30 Tage durch Verabreichung der erfindungsgemäßen magensäurestabilen Kapseln behandelt (Dosierung: 2 Kapseln täglich). Bei jeder Person wurden zu Beginn der Untersuchung (vor der Verabreichung der ersten Kapsel, Spalte "Vor" in der nachstehenden Tabelle 2) und am Tag 30 der Untersuchung (Spalte "Nach" in der nachstehenden Tabelle 2) die Blutkonzentrationen von Ferritin, IL6 und Hämoglobin (Hb) gemessen. Die Ergebnisse und deren prozentuale Veränderungen sind in der Tabelle 2 gezeigt.

**Tabelle 2: Untersuchungsergebnisse und deren prozentuale Veränderung**

| Patient Nr. | Alter (Gesc hlecht) | Ferritin (ng/ml) | | | IL6 (pg/ml) | | | Hb (mg/dl) | | |
|---|---|---|---|---|---|---|---|---|---|---|
| | | Vor | Nach | % Veränd. | Vor | Nach | % Veränd. | Vor | Nach | % Veränd. |
| 1 | 91 (w) | 8 | 60 | 650 | 90 | 80 | -11 | 11,6 | 12,1 | 4,3 |
| 2 | 74 (m) | 19 | 117 | 516 | 80 | 48 | -40 | 13,2 | 14,0 | 6,1 |
| 3 | 85 (w) | 10 | 90 | 800 | 42 | 35 | -17 | 11,5 | 12,2 | 6,1 |
| 4 | 54 (m) | 7 | 78 | 1014 | 55 | 45 | -18 | 13,2 | 13,6 | 3,0 |
| 5 | 71 (m) | 29 | 105 | 262 | 78 | 58 | -26 | 9,2 | 9,5 | 3,3 |

Wie es aus der Tabelle 2 ersichtlich ist, waren nach 30 Tagen der Verabreichung der erfindungsgemäßen magensäurestabilen Kapseln die Konzentrationen von Ferritin und Hb bei den Patienten sehr stark bzw. stark angestiegen, und die IL6-Konzentrationen haben durchweg abgenommen. Dies zeigt deutlich die positive Wirkung der erfindungsgemäßen magensäurestabilen Kapseln auf die Konzentration von Eisen im Blut der untersuchten Patienten.

Die unter Bezug auf die dargestellte Ausführungsform beschriebenen Merkmale der Erfindung, wie beispielsweise das Verhältnis der Bestandteile in der Formulierungszusammensetzung, das für die magensäurestabile Kapsel angegeben ist, können auch bei anderen Ausführungsformen der Erfindung vorhanden sein, wie beispielsweise anderen möglichen Formen, in welche die Formulierungszusammensetzung gebracht werden kann, wie z.B. Tabletten mit verzögerter Freisetzung, außer wenn es anders angegeben ist oder sich aus technischen Gründen von selbst verbietet.

## Patentansprüche

1. Formulierung, die
Lactoferrin,
Eisenverbindungen und
Vitamin C umfasst.

2. Formulierung nach Anspruch 1, bei der das Lactoferrin aus Kuhmilch oder Molke gewonnen worden ist.

3. Formulierung nach Anspruch 1 oder 2, bei der das Lactoferrin, bezogen auf 100 Gew.-% des Lactoferrins, weniger als 0.00001, also 10^{∗}10^-6, Gew.-% Lactoperoxidase, weniger als 0.00001, also 10^{∗}10^-6, Gew.-% Angiogenin, und weniger als 0.00001, also 10^{∗}10^-6, Gew.-% Lipopolysaccharide umfasst.

4. Formulierung nach einem der Ansprüche 1 bis 3, bei der die Eisenverbindungen pflanzliche Eisenverbindungen sind.

5. Formulierung nach Anspruch 4, bei der die pflanzlichen Eisenverbindungen in der Form eines Curryblattextrakts vorliegen.

6. Formulierung nach einem der Ansprüche 1 bis 5, bei der das Vitamin C in der Form eines Hagebuttenextrakts vorliegt.

7. Formulierung nach einem der Ansprüche 1 bis 6,
die, bezogen auf 100 Gew.-% der Formulierung,
70 bis 90 Gew.-% Lactoferrin,
1,5 bis 3,5 Gew.-% Eisenverbindungen und
6,5 bis 28,5 Gew.-% Vitamin C umfasst.

8. Formulierung nach Anspruch 6, soweit dieser auf Anspruch 5 rückbezogen ist, die, bezogen auf 100 Gew.-% der Formulierung,
20 bis 30 Gew.-% Lactoferrin,
55 bis 75 Gew.-% Curryblattextrakt und
5 bis 15 Gew-% Hagebuttenextrakt umfasst.

9. Ernährungsphysiologische Zusammensetzung, die eine Formulierung nach einem der Ansprüche 1 bis 8 und einen oder mehrere pharmazeutisch verträgliche(n) Hilfsstoff(e) umfasst.

10. Magensäurestabile Kapsel, die eine Formulierung nach einem der Ansprüche 1 bis 8 oder eine ernährungsphysiologische Zusammensetzung nach Anspruch 9 umfasst.

11. Verwendung einer Formulierung nach einem der Ansprüche 1 bis 8 oder einer ernährungsphysiologischen Zusammensetzung nach Anspruch 9 für eine ergänzend bilanzierte Diät zur Anwendung bei einem erhöhten Eisenbedarf bei entzündlichen Darmerkrankungen und Unverträglichkeit von Eisensalzen.
